(19) **Europäisches Patentamt / European Patent Office / Office européen des brevets**

(11) **EP 4 721 664 A1**

(12) # EUROPEAN PATENT APPLICATION

(43) Date of publication:
08.04.2026 Bulletin 2026/15

(21) Application number: 25205530.6

(22) Date of filing: 30.09.2025

(51) International Patent Classification (IPC):
*A61B 5/287* (2021.01)   *A61B 5/361* (2021.01)
*A61B 5/00* (2006.01)

(52) Cooperative Patent Classification (CPC):
A61B 5/287; A61B 5/361; A61B 5/743

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: 01.10.2024 US 202418903377

(71) Applicant: **Biosense Webster (Israel) Ltd.**
**2066717 Yokneam (IL)**

(72) Inventors:
• **RODRIGUEZ, Haim**
  **2066717 Yokneam (IL)**
• **AMIT, Matityahu**
  **2066717 Yokneam (IL)**

(74) Representative: **Carpmaels & Ransford LLP**
**One Southampton Row**
**London WC1B 5HA (GB)**

(54) **IDENTIFICATION OF SITES CORRESPONDING TO SUSPECTED ATRIAL FIBRILLATION TRIGGERS BASED ON CORRELATION OF SIGNALS FROM MULTIPLE ELECTRODES**

(57)   A composite correlation score associated with a selected spatial location in the heart is determined based on a plurality of individual correlation values, each of which is based on a correlation between a signal associated with a selected electrode positioned at the selected spatial location and a signal associated with another one of the plurality of electrodes when the selected electrode is positioned at the selected spatial location. Composite correlation scores associated other selected spatial locations in the heart are determined the same way. An electro-anatomical map of the heart is displayed which depicts information representative of the composite correlation score determined for the selected spatial locations, including information indicating that a spatial location is a suspected trigger of AF.

EP 4 721 664 A1

# EP 4 721 664 A1

**Description**

FIELD OF INVENTION

The present invention is related to anatomical mapping. More particularly, the present invention relates to improving the visualization of spatial locations in an electro-anatomical map of a heart representing suspected triggers of atrial fibrillation.

BACKGROUND

Atrial fibrillation (AF) occurs when the electrical signals in the heart become disorganized, leading to an irregular and often rapid heartbeat. This can be due to abnormal electrical activity in the heart's atria, the upper chambers of the heart. Ablation is a medical procedure used to treat AF. The goal of ablation is to restore normal heart rhythm by targeting and disrupting abnormal electrical signals causing the AF.

In catheter ablation, a catheter is inserted through a blood vessel, usually in the groin, and threaded up to the heart. Once in place, the catheter delivers energy to specific areas of the heart tissue. This energy ablates the tissue and disrupts the abnormal electrical signals, helping to restore a normal rhythm.

Some catheter ablation procedures employ techniques for generation and analysis of a computerized anatomical map of the heart. For example, in electrophysiology (EP) procedures such as catheter-based radio frequency (RF) ablation, an anatomical map of a heart chamber is generated and used. In connection with catheter ablation procedures that employ a computerized anatomical map of the heart, a need exists for automated and more accurate techniques that identify spatial locations on the anatomical map that correspond to suspected triggers of AF as candidates for ablation during the procedure.

SUMMARY

A technique is disclosed for identifying one or more spatial locations that correspond to suspected triggers of AF in a human heart. The technique is performed during an ablation procedure using a catheter having a plurality of electrodes positioned in the heart. The electrodes collect electrical signals from spatial locations within the heart as the catheter moves within the heart during the procedure. According to the technique, at least one composite correlation score associated with a selected spatial location in the heart is determined. The at least one composite correlation score is based on a plurality of individual correlation values, wherein each individual correlation value is based on a correlation between at least one signal associated with a selected electrode positioned at the selected spatial location and at least one signal associated with another one of the plurality of electrodes when the selected electrode is positioned at the selected spatial location. At least one composite correlation score associated with each of a plurality of other selected spatial locations in the heart is determined the same way for each of the other selected spatial locations. An electro-anatomical map of the heart is displayed, wherein the map spatially depicts information representative of the composite correlation score determined for each of the selected spatial locations. The information representative of the composite correlation score displayed on the electro-anatomical map for at least one of the selected spatial locations indicates that the at least one selected spatial location corresponds to a suspected trigger of AF.

In some examples, each individual correlation value is based on a correlation between a first frequency modulated signal associated with the selected electrode positioned at the selected spatial location and a second frequency modulated signal associated with another one of the plurality of electrodes when the selected electrode is positioned at the selected spatial location.

In some examples, the at least one composite correlation score corresponds to an average of the plurality of individual correlation values.

In some examples, each individual correlation value is also based on a correlation between a first amplitude modulated signal associated with the selected electrode positioned at the selected spatial location and a second amplitude modulated signal associated with another one of the plurality of electrodes when the selected electrode is positioned at the selected spatial location.

In some examples, two electro-anatomical maps of the heart are displayed (e.g., side-by side). The first map spatially depicts information representative of a first composite correlation score determined for each of the selected spatial locations; wherein the first composite correlation score for each of the selected spatial locations is based on a plurality of first individual correlation values each of which is based on a correlation between frequency modulated signals. The second map spatially depicts information representative of a second composite correlation score determined for each of the selected spatial locations; wherein the second composite correlation score for each of the selected spatial locations is based on a plurality of second individual correlation values each of which is based on a correlation between amplitude modulated signals.

In some examples, the at least one signal associated with the selected electrode positioned at the selected spatial location is generated by collecting a first electrical signal from the selected electrode, and applying at least far field reduction processing and band pass filtering to the first electrical signal.

In some examples, the at least one signal associated with the selected electrode positioned at the selected spatial location is generated by collecting a first electrical signal from the selected electrode, applying signal processing to the first electrical signal to generate a second electrical signal, and converting the second electrical signal into a binary signal.

In some examples, each individual correlation value is based on a linear correlation between the at least one signal derived from the selected electrode positioned at the selected spatial location and the at least one signal derived from another one of the plurality of electrodes when the selected electrode is positioned at the selected spatial location.

In some examples, the information representative of the composite correlation score displayed on the electro-anatomical map for at least one of the selected spatial locations indicates that the at least one selected spatial location corresponds to a suspected trigger of AF if the spatial position has a corresponding composite correlation score that is above a threshold.

According to one or more embodiments, the exemplary embodiments above can be implemented as methods, apparatuses, systems, and/or computer program products.

BRIEF DESCRIPTION OF THE DRAWINGS

A more detailed understanding may be had from the following description, given by way of example in conjunction with the accompanying drawings, wherein like reference numerals in the figures indicate like elements, and wherein:

FIG. 1 depicts an example catheter-based electrophysiology mapping and ablation system according to one or more embodiments;

FIG. 2 is a block diagram of an example system for remotely monitoring and communicating biometric data according to one or more embodiments;

FIG. 3 is a system diagram of an example computing environment in communication with a network according to one or more embodiments;

FIG. 4A is a block diagram of an example device in which one or more features of the disclosure can be implemented according to one or more embodiments;

FIG. 4B shows an example of a linear catheter including multiple electrodes that may be used to map a cardiac area;

FIG. 5 shows an example of a balloon catheter including multiple splines (e.g., 12 splines in the specific example of FIG. 5) including splines and multiple electrodes on each spline including electrodes;

FIG. 6 shows an example of a loop catheter (also referred to as a lasso catheter) including multiple electrodes that may be used to map a cardiac area;

FIG. 7 shows an example of a flat type multi-electrode catheter that may be used to map a cardiac area, in accordance with an example;

FIG. 8 depicts an embodiment of a technique for generating composite correlation scores based on electrical signals from a multi-electrode catheter, in accordance with an example;

FIG. 9 depicts an embodiment of a technique for generating composite correlation scores based on electrical signals from a multi-electrode catheter, in accordance with a further example;

FIG. 10 depicts an embodiment of a technique for generating composite correlation scores based on electrical signals from a multi-electrode catheter, in accordance with a still further example;

Figure 11 illustrates generation of a binary signal based on a frequency modulated signal associated with a catheter electrode, according to an example;

Figure 12 illustrates generation of a binary signal based on an amplitude modulated signal associated with a catheter electrode, according to an example;

Figure 13 illustrates a technique for identifying an individual correlation value based on frequency modulated signals associated with two different electrodes of a multi-catheter electrode, according to an example;

Figure 14 illustrates examples of electro-anatomical maps depicting composite correlation scores determined for multiple spatial locations in a heart, according to examples; and

FIG. 15 depicts a method according to one or more embodiments.

DETAILED DESCRIPTION

Disclosed herein is a method and/or system for identifying one or more spatial locations that correspond to suspected triggers of AF in a human heart and displaying the identified spatial locations as part of an electro-anatomical mapping of the heart. The method and/or system includes a processor executable code or software that is necessarily rooted in process operations by, and in processing hardware of, medical device equipment performing and using the anatomical mapping.

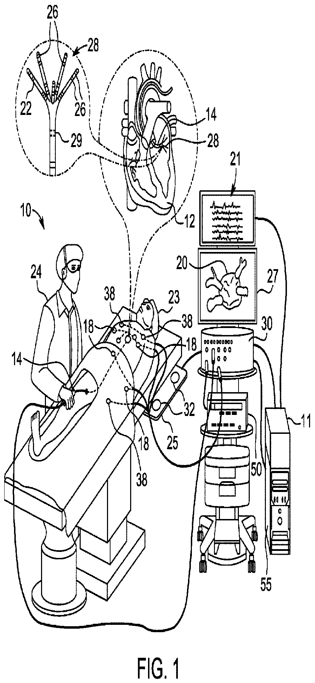

Reference is made to FIG. 1 showing an example system (e.g., medical device equipment and/or catheter-based electrophysiology mapping and ablation system), shown as system 10, in which one or more features of the subject matter herein can be implemented according to one or more embodiments. All or part of the system 100 can be used to collect information (e.g., biometric data) and/or used to implement the techniques as described herein. In some examples, such techniques are implemented using a processor executable code or software that is stored on a memory of the system 10 and that is necessarily rooted in process operations by, and in processing hardware of, the system 10.

FIG. 1 illustrates a recorder 11, a heart 12, a catheter 14, a model or anatomical map 20, an electrogram 21, a spline 22, a patient 23, a physician 24 (which is representative of any medical professional, technician, clinician, operator, clinical support specialist, clinical account specialist, healthcare personnel, etc.), a location pad 25, one or more electrodes 26, a display device 27, a distal tip 28, a sensor 29, a coil 32, a patient interface unit (PIU) 30, electrode skin patches 38, an ablation energy generator 50, and a workstation 55. Note further each element and/or item of the system 10 is representative of one or more of that element and/or that item. The example system 10 shown in FIG. 1 implements the embodiments disclosed herein. The disclosed embodiments can similarly be applied using other system components and settings. Additionally, the system 10 can include additional components, for example elements for sensing electrical activity, wired or wireless connectors, processing and display devices, or other components.

The system 10 includes multiple catheters 14, which are percutaneously inserted by the physician 24 through the patient's vascular system into a chamber or vascular structure of the heart 12. Typically, a delivery sheath catheter is inserted into the left or right atrium near a desired location in the heart 12. Thereafter, a plurality of catheters can be inserted into the delivery sheath catheter to arrive at the desired location. The plurality of catheters 14 may include catheters dedicated for sensing Intracardiac Electrogram (IEGM) signals, catheters dedicated for ablating, and/or catheters dedicated for both sensing and ablating. The example catheter 14 that is configured for sensing IEGM is illustrated herein. The physician 24 brings the distal tip 28 of the catheter 14 into contact with a heart wall for sensing a target site in the heart 12. For ablation, the physician 24 would similarly bring a distal end of an ablation catheter to a target site for ablating.

The catheter 14 is an exemplary catheter that includes multiple electrodes 26 optionally distributed over a plurality of splines 22 at the distal tip 28 and configured to sense the IEGM signals. The catheter 14 may additionally include the sensor 29 embedded in or near the distal tip 28 for tracking position and orientation of the distal tip 28. Optionally and preferably, the position sensor 29 is a magnetic based position sensor including three magnetic coils for sensing 3D position and orientation. According to one or more embodiments, shape and parameters of the catheter 14 vary based on whether the catheter 14 is used for diagnostic or ablation purposes, the type of arrhythmia, patient anatomy, and other factors, which affect catheter maneuverability (e.g., an ability to touch without bending the surface and the tracked parts of the catheter 14). The shape and parameters of the catheter 14 also impact the accuracy of anatomical maps. Large spherical single-shot catheters, which can ablate a pulmonary vein within seconds, have become popular but require guidance from fluoroscopy, CT/MRI, or additional mapping catheters.

The sensor 29 (e.g., a position or a magnetic based position sensor) may be operated together with the location pad 25 including a plurality of magnetic coils 32 configured to generate magnetic fields in a predefined working volume. Real time position of the distal tip 28 of the catheter 14 may be tracked based on magnetic fields generated with the location pad 25 and sensed by the sensor 29. Details of the magnetic based position sensing technology are described in U.S. Patent Nos. 5,5391,199; 5,443,489; 5,558,091; 6,172,499; 6,239,724; 6,332,089; 6,484,118; 6,618,612; 6,690,963; 6,788,967; 6,892,091.

The system 10 includes one or more electrode patches 38 positioned for skin contact on the patient 23 to establish location reference for the location pad 25 as well as impedance-based tracking of the electrodes 26. For impedance-based tracking, electrical current is directed toward the electrodes 26 and sensed at the patches 38 (e.g., electrode skin patches) so that the location of each electrode can be triangulated via the patches 38. Details of the impedance-based location tracking technology are described in US Patent Nos. 7,536,218; 7,756,576; 7,848,787; 7,869,865; and 8,456,182, which are incorporated herein by reference.

The recorder 11 displays the electrograms 21 captured with the electrodes 18 (e.g., body surface electrocardiogram (ECG) electrodes) and intracardiac electrograms (IEGM) captured with the electrodes 26 of the catheter 14. The recorder 11 may include pacing capability for pacing the heart rhythm and/or may be electrically connected to a standalone pacer.

The system 10 may include the ablation energy generator 50 that is adapted to conduct ablative energy to the one or more of electrodes 26 at the distal tip 28 of the catheter 14 configured for ablating. Energy produced by the ablation energy generator 50 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

The PIU 30 is an interface configured to establish electrical communication between catheters, electrophysiological equipment, power supply and the workstation 55 for controlling operation of the system 10. Electrophysiological equipment of the system 10 may include for example, multiple catheters 14, the location pad 25, the body surface ECG electrodes 18, the electrode patches 38, the ablation energy generator 50, and the recorder 11. Optionally and preferably, the PIU 30 additionally includes processing capability for implementing real-time computations of location of the catheters and for performing ECG calculations.

The workstation 55 includes memory, a processor unit with memory or storage with appropriate operating software loaded therein, and user interface capability. The workstation 55 may provide multiple functions, optionally including modeling the endocardial anatomy in three-dimensions (3D) and rendering the model or anatomical map 20 (e.g., a visualization) for display on the display device 27, displaying on the display device 27 activation sequences (or other data) compiled from recorded electrograms 21 in representative visual indicia or imagery superimposed on the rendered anatomical map 20, displaying real-time location and orientation of multiple catheters within the heart chamber, and displaying on the display device 27 sites of interest for example places where ablation energy has been applied. In some examples discussed below, the rendered electro-anatomical map spatially depicts information representative of a composite correlation score determined for selected spatial locations, such information being indicative of one or more spatial locations corresponding to suspected trigger(s) of AF. One commercial product embodying elements of the system 10 is available as the CARTO™ 3 System, available from Biosense Webster, Inc., 31A Technology Drive, Irvine, CA 92618. Note that modeling the endocardial anatomy in 3D can include generating a surface thereof as a triangular mesh.

For instance, the system 10 can be part of a surgical system (e.g., CARTO® system sold by Biosense Webster) that is configured to obtain biometric data (e.g., anatomical and electrical measurements of a patient's organ, for example the heart 12 and as described herein) and perform a cardiac ablation procedure. More particularly, treatments for cardiac conditions for example cardiac arrhythmia often require obtaining a detailed mapping of cardiac tissue, chambers, veins, arteries and/or electrical pathways. For example, a prerequisite for performing a catheter ablation successfully is that the cause of the cardiac arrhythmia is accurately located in a chamber of the heart 12. Such locating may be done via an electrophysiological investigation during which electrical potentials are detected and spatially resolved with a mapping catheter (e.g., the catheter 14) introduced into the chamber of the heart 12. This electrophysiological investigation, the so-called electro-anatomical mapping, thus provides 3D mapping data which can be displayed on the display device 27. In many cases, the mapping function and a treatment function (e.g., ablation) are provided by a single catheter or group of catheters such that the mapping catheter also operates as a treatment catheter at the same time.

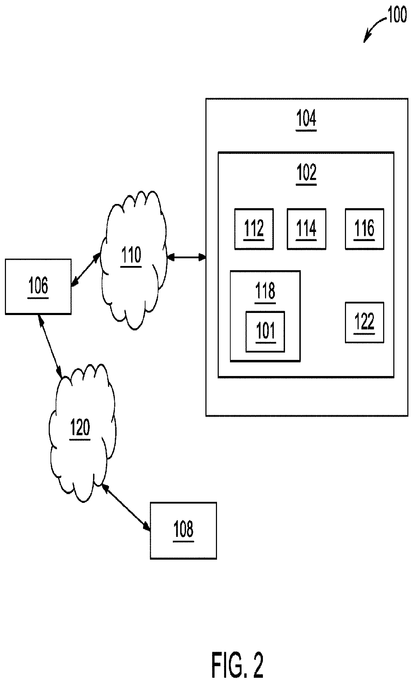

FIG. 2 is a block diagram of an example system 100 for remotely monitoring and communicating biometric data (e.g., patient biometrics). In the example illustrated in FIG. 2, the system 100 includes a patient biometric monitoring and processing apparatus 102 associated with a patient 104, a local computing device 106, a remote computing system 108, a first network 110, a patient biometric sensor 112, a processor 114, a user input (UI) sensor 116, a memory 118, a second network 120, and a transmitter-receiver (i.e., transceiver) 122.

According to one or more embodiments, the patient biometric monitoring and processing apparatus 102 may be an apparatus that is internal to the patient's body (e.g., subcutaneously implantable), for example the catheter 14 of FIG. 1. The patient biometric monitoring and processing apparatus 102 may be inserted into a patient via any applicable manner including orally injecting, surgical insertion via a vein or artery, an endoscopic procedure, or a laparoscopic procedure.

According to one or more embodiments, the patient biometric monitoring and processing apparatus 102 may be an apparatus that is external to the patient, for example the electrode patches 38 of FIG. 1. For example, as described in more detail below, the patient biometric monitoring and processing apparatus 102 may include an attachable patch (e.g., that attaches to a patient's skin). The monitoring and processing apparatus 102 may also include a catheter with one or more electrodes, a probe, a blood pressure cuff, a weight scale, a bracelet or smart watch biometric tracker, a glucose monitor, a continuous positive airway pressure (CPAP) machine or virtually any device which may provide an input concerning the health or biometrics of the patient.

According to one or more embodiments, the patient biometric monitoring and processing apparatus 102 may include both components that are internal to the patient and components that are external to the patient.

A single patient biometric monitoring and processing apparatus 102 is shown in FIG. 2. Example systems may, however, include a plurality of patient biometric monitoring and processing apparatuses. A patient biometric monitoring and processing apparatus may be in communication with one or more other patient biometric monitoring and processing apparatuses. Additionally or alternatively, a patient biometric monitoring and processing apparatus may be in communication with the network 110.

One or more patient biometric monitoring and processing apparatuses 102 may acquire biometric data (e.g., patient biometrics, for example electrical signals, blood pressure, temperature, blood glucose level, or other biometric data) and receive at least a portion of the biometric data representing the acquired patient biometrics and additional information associated with the acquired patient biometrics from one or more other patient biometric monitoring and processing apparatuses 102. The additional information may be, for example, diagnosis information and/or additional information obtained from an additional device, for example a wearable device. Each of the patient biometric monitoring and processing apparatus 102 may process data, including its own biometric data as well as data received from one or more other patient biometric monitoring and processing apparatuses 102.

Biometric data (e.g., patient biometrics, patient data, or patient biometric data) can include one or more of local activation times (LATs), electrical activity, topology, bipolar mapping, reference activity, ventricle activity, dominant frequency, impedance, or other data. The LAT can be a point in time of a threshold activity corresponding to a local activation, calculated based on a normalized initial starting point. Electrical activity can be any applicable electrical signals that can be measured based on one or more thresholds and can be sensed and/or augmented based on signal to noise ratios and/or other filters. A topology can correspond to the physical structure of a body part or a portion of a body part and can correspond to changes in the physical structure relative to different parts of the body part or relative to different body parts. A dominant frequency can be a frequency or a range of frequency that is prevalent at a portion of a body part and can be different in different portions of the same body part. For example, the dominant frequency of a PV of a heart can be different than the dominant frequency of the right atrium of the same heart. Impedance can be the resistance measurement at a given area of a body part.

Examples of biometric data include, but are not limited to, patient identification data, intracardiac electrocardiogram (IC ECG) data, bipolar intracardiac reference signals, anatomical and electrical measurements, trajectory information, body surface (BS) ECG data, historical data, brain biometrics, blood pressure data, ultrasound signals, radio signals, audio signals, two- or three-dimensional image data, blood glucose data, and temperature data. The biometrics data can be used, generally, to monitor, diagnosis, and treat any number of various diseases, for example cardiovascular diseases (e.g., arrhythmias, cardiomyopathy, and coronary artery disease) and autoimmune diseases (e.g., type I and type II diabetes). Note that BS ECG data can include data and signals collected from electrodes on a surface of a patient, IC ECG data can include data and signals collected from electrodes within the patient, and ablation data can include data and signals collected from tissue that has been ablated. Further, BS ECG data, IC ECG data, and ablation data, along with catheter electrode position data, can be derived from one or more procedure recordings.

In FIG. 2, the network 110 is an example of a short-range network (e.g., local area network (LAN), or personal area network (PAN)). Information may be sent, via the network 110, between the patient biometric monitoring and processing apparatus 102 and the local computing device 106 using any one of various short-range wireless communication protocols, for example Bluetooth, Wi-Fi, Zigbee, Z-Wave, near field communications (NFC), ultraband, or infrared (IR).

The network 120 may be a wired network, a wireless network or include one or more wired and wireless networks. For example, the network 120 may be a long-range network (e.g., wide area network (WAN), the internet, or a cellular network). Information may be sent, via the network 120 using any one of various long-range wireless communication protocols (e.g., TCP/IP, HTTP, 3G, 4G/LTE, or 5G/New Radio).

The patient biometric monitoring and processing apparatus 102 may include the patient biometric sensor 112, the processor 114, the UI sensor 116, the memory 118, and the transceiver 122. The patient biometric monitoring and processing apparatus 102 may continually or periodically monitor, store, process and communicate, via the network 110, any number of various biometric data. Examples of biometric data include electrical signals (e.g., ECG signals and brain biometrics), blood pressure data, blood glucose data, and temperature data. The biometric data may be monitored and communicated for treatment across any number of various diseases, for example cardiovascular diseases (e.g., arrhythmias, cardiomyopathy, and coronary artery disease) and autoimmune diseases (e.g., type I and type II diabetes).

The patient biometric sensor 112 may include, for example, one or more sensors configured to sense a type of biometric data. For example, the patient biometric sensor 112 may include an electrode configured to acquire electrical signals (e.g., heart signals, brain signals or other bioelectrical signals), a temperature sensor, a blood pressure sensor, a blood glucose sensor, a blood oxygen sensor, a pH sensor, an accelerometer and a microphone.

As described in more detail below, the patient biometric monitoring and processing apparatus 102 may be an ECG monitor for monitoring ECG signals of a heart (e.g., the heart 12). The patient biometric sensor 112 of the ECG monitor may include one or more electrodes for acquiring ECG signals. The ECG signals may be used for treatment of various cardiovascular diseases, as well as anatomical mapping.

The transceiver 122 may include a separate transmitter and receiver. Alternatively, the transceiver 122 may include a transmitter and receiver integrated into a single device.

The processor 114 may be configured to store biometric data in the memory 118 acquired by the patient biometric sensor 112 and to communicate the biometric data across the network 110 via a transmitter of the transceiver 122. Data from one or more other patient biometric monitoring and processing apparatus 102 may also be received by a receiver of the transceiver 122, as described in more detail herein. By way of example, the techniques for identifying one or more spatial locations that correspond to suspected triggers of AF in a heart described herein are implemented as a processor executable code or software that can be stored the memory 118 (as shown) and executed by the processor 114. By way of further example, the techniques for identifying one or more spatial locations corresponding to suspected triggers of AF are implemented as code stored and executed on the local computing device 106 and/or the remote computing system 108. Thus, the operation of the techniques for identifying one or more spatial locations that correspond to suspected triggers of AF are necessarily rooted in process operations by, and in processing hardware of, the system 100.

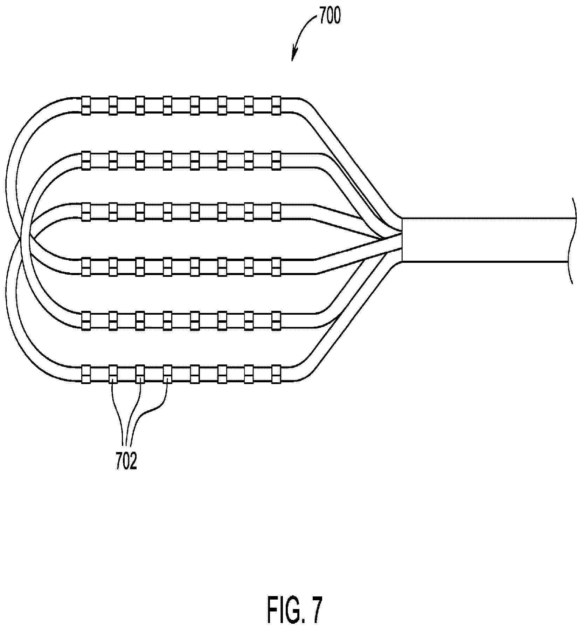

According to one or more embodiments, system 100 operates during an ablation procedure to identify one or more spatial locations on an electro-anatomical map depicted on display device 27 that correspond to suspected triggers of AF in a human heart. Applying the techniques described in connection with FIGs. 7-14 below, the electrodes collect electrical signals from spatial locations within the heart as a multi-electrode catheter moves within the heart during the procedure. A composite correlation score associated with a selected spatial location in the heart is determined based on a plurality of individual correlation values, each of which is based on a correlation between a signal (e.g., an FM signal) associated with a selected electrode positioned at the selected spatial location and a signal (e.g., another FM signal) associated with another one of the plurality of electrodes when the selected electrode is positioned at the selected spatial location. A composite correlation score associated with each of a plurality of other selected spatial locations in the heart is determined the same way for each of the other selected spatial locations. An electro-anatomical map of the heart is displayed (e.g., on display 27) spatially depicting information representative of the composite correlation score determined for each of the selected spatial locations, where one or more spatial locations on the map correspond to a suspected trigger of AF. In some examples, the composite correlation score corresponds to an average of the plurality of individual correlation values.

According to one or more embodiments, the patient biometric monitoring and processing apparatus 102 includes UI sensor 116 that may be, for example, a piezoelectric sensor or a capacitive sensor configured to receive a user input, for example a tapping or touching. For example, the UI sensor 116 may be controlled to implement a capacitive coupling, in response to tapping or touching a surface of the patient biometric monitoring and processing apparatus 102 by the patient 104. Gesture recognition may be implemented via any one of various capacitive types, for example resistive capacitive, surface capacitive, projected capacitive, surface acoustic wave, piezoelectric and infrared touching. Capacitive sensors may be disposed at a small area or over a length of the surface such that the tapping or touching of the surface activates the monitoring device.

As described in more detail below, the processor 114 may be configured to respond selectively to different tapping patterns of the capacitive sensor (e.g., a single tap or a double tap), which may be the UI sensor 116, such that different tasks of the patch (e.g., acquisition, storing, or transmission of data) may be activated based on the detected pattern. In some embodiments, audible feedback may be given to the user from the patient biometric monitoring and processing apparatus 102 when a gesture is detected.

The local computing device 106 of the system 100 is in communication with the patient biometric monitoring and processing apparatus 102 and may be configured to act as a gateway to the remote computing system 108 through the second network 120. The local computing device 106 may be, for example, a, smart phone, smartwatch, tablet or other portable smart device configured to communicate with other devices via the network 120. Alternatively, the local computing device 106 may be a stationary or standalone device, for example a stationary base station including, for example, modem and/or router capability, a desktop or laptop computer using an executable program to communicate information between the patient biometric monitoring and processing apparatus 102 and the remote computing system 108 via the PC's radio module, or a USB dongle. Biometric data may be communicated between the local computing device 106 and the patient biometric monitoring and processing apparatus 102 using a short-range wireless technology standard (e.g., Bluetooth, Wi-Fi, ZigBee, Z-wave and other short-range wireless standards) via the short-range wireless network 110, for example a local area network (LAN) (e.g., a personal area network (PAN)). In some embodiments, the local computing device 106 may also be configured to display the acquired patient electrical signals and information associated with the acquired patient electrical signals, as described in more detail herein.

In some embodiments, the remote computing system 108 may be configured to receive at least one of the monitored patient biometrics and information associated with the monitored patient via network 120, which is a long-range network. For example, if the local computing device 106 is a mobile phone, network 120 may be a wireless cellular network, and information may be communicated between the local computing device 106 and the remote computing system 108 via a wireless technology standard, for example any of the wireless technologies mentioned above. As described in more detail below, the remote computing system 108 may be configured to provide (e.g., visually display and/or aurally provide) the at least one of the patient biometrics and the associated information to the physician 24.

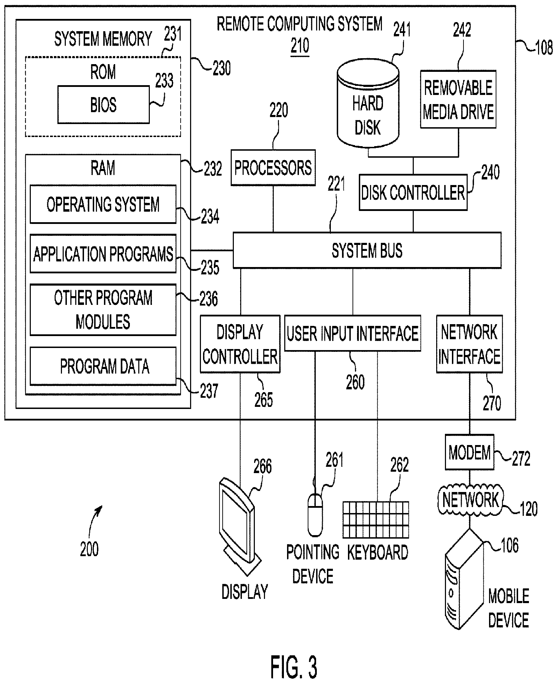

FIG. 3 is a system diagram of an example of a computing environment 200 in communication with network 120. In some instances, the computing environment 200 is incorporated in a public cloud computing platform (e.g., Amazon Web Services or Microsoft Azure), a hybrid cloud computing platform (e.g., HP Enterprise OneSphere) or a private cloud computing platform.

As shown in FIG. 3, computing environment 200 includes a computer system 210, which is one example of the workstation 55 of FIG. 1, the local computing device 106 of FIG. 2, and/or the remote computing system 108 of FIG. 2 upon which embodiments described herein may be implemented. By way of example, techniques for identification of spatial locations corresponding to suspected AF triggers described herein are implemented as a processor executable code or software that can be stored on the system memory 231 (as shown) and executed by processors 220, and rooted in process operations by, and in processing hardware of, the computing environment 200.

The computer system 210 may, via processors 220, which may include one or more processors, perform various functions. The functions may include analyzing monitored biometric data and the associated information and, according to physician-determined or algorithm driven thresholds and parameters, providing (e.g., via display 266) alerts, additional information or instructions. The functions may include the operation of the techniques for identification of spatial locations corresponding to suspected AF triggers described herein. As described in more detail herein, the computer system 210 may be used to provide (e.g., via display 266) the physician 24 of FIG. 1 with a dashboard of patient information, such that such information may enable the physician 24 to identify and prioritize patients having more critical needs than others.

As shown in FIG. 3, the computer system 210 may include a communication mechanism, for example a bus 221 or other communication mechanism for communicating information within the computer system 210. The computer system 210 further includes one or more processors 220 coupled with the bus 221 for processing the information. The processors 220 may include one or more CPUs, GPUs, or any other processor known in the art.

The computer system 210 also includes a system memory 230 coupled to the bus 221 for storing information and instructions to be executed by processors 220. The system memory 230 may include computer readable storage media in the form of volatile and/or nonvolatile memory, for example read only system memory (ROM) 231 and/or random-access memory (RAM) 232. The system memory RAM 232 may include other dynamic storage device(s) (e.g., dynamic RAM, static RAM, and synchronous DRAM). The system memory ROM 231 may include other static storage device(s) (e.g., programmable ROM, erasable PROM, and electrically erasable PROM). In addition, the system memory 230 may be used for storing temporary variables or other intermediate information during the execution of instructions by the processors 220. A basic input/output system 233 (BIOS) may contain routines to transfer information between elements within computer system 210, for example during start-up, that may be stored in system memory ROM 231. RAM 232 may comprise data and/or program modules that are immediately accessible to and/or presently being operated on by the processors 220. System memory 230 may additionally include, for example, operating system 234, application programs 235, other program modules 236 and program data 237.

The illustrated computer system 210 also includes a disk controller 240 coupled to the bus 221 to control one or more storage devices for storing information and instructions, for example a magnetic hard disk 241 and a removable media drive 242 (e.g., floppy disk drive, compact disc drive, tape drive, and/or solid-state drive). The storage devices may be added to the computer system 210 using an appropriate device interface (e.g., a small computer system interface (SCSI), integrated device electronics (IDE), Universal Serial Bus (USB), or FireWire).

The computer system 210 may also include a display controller 265 coupled to the bus 221 to control a monitor or display 266, for example a cathode ray tube (CRT) or liquid crystal display (LCD), for displaying information to a computer user. The illustrated computer system 210 includes a user input interface 260 and one or more input devices, for example a keyboard 262 and a pointing device 261, for interacting with a computer user and providing information to the processor 220. The pointing device 261, for example, may be a mouse, a trackball, or a pointing stick for communicating direction information and command selections to the processor 220 and for controlling cursor movement on the display 266. The display 266 may provide a touch screen interface that may allow input to supplement or replace the communication of direction information and command selections by the pointing device 261 and/or keyboard 262.

The computer system 210 may perform a portion or each of the functions and methods described herein in response to the processors 220 executing one or more sequences of one or more instructions contained in a memory, for example, the system memory 230. Such instructions may be read into the system memory 230 from another computer readable medium, for example, a hard disk 241 or a removable media drive 242. The hard disk 241 may contain one or more data stores and data files used by embodiments described herein. Data store contents and data files may be encrypted to improve security. The processors 220 may also be employed in a multi-processing arrangement to execute the one or more sequences of instructions contained in system memory 230. In alternative embodiments, hard-wired circuitry may be used in place of or in combination with software instructions. Thus, embodiments are not limited to any specific combination of hardware circuitry and software.

As stated above, the computer system 210 may include at least one computer readable medium or memory for holding instructions programmed according to embodiments described herein (e.g., embodiments of the tenting error detection and correction techniques) and for containing data structures, tables, records, or other data described herein. The term computer readable medium as used herein refers to any non-transitory, tangible medium that participates in providing instructions to the processor 220 for execution. A computer readable medium may take many forms including, but not limited to, non-volatile media, volatile media, and transmission media. Non-limiting examples of non-volatile media include optical disks, solid state drives, magnetic disks, and magneto-optical disks, for example hard disk 241 or removable media drive 242. Non-limiting examples of volatile media include dynamic memory, for example system memory 230. Non-limiting examples of transmission media include coaxial cables, copper wire, and fiber optics, including the wires that make up the bus 221. Transmission media may also take the form of acoustic or light waves, for example those generated during radio wave and infrared data communications.

The computing environment 200 may further include the computer system 210 operating in a networked environment using logical connections to local computing device 106 and one or more other devices, for example a personal computer (laptop or desktop), mobile devices (e.g., patient mobile devices), a server, a router, a network PC, a peer device or other common network node, and typically includes many or all of the elements described above relative to computer system 210. When used in a networking environment, computer system 210 may include modem 272 for establishing communications over a network 120, for example the Internet. Modem 272 may be connected to system bus 221 via network interface 270, or via another appropriate mechanism.

Network 120, as shown in FIGS. 2 and 3, may be any network or system generally known in the art, including the Internet, an intranet, a local area network (LAN), a wide area network (WAN), a metropolitan area network (MAN), a direct connection or series of connections, a cellular telephone network, or any other network or medium capable of facilitating communication between computer system 210 and other computers (e.g., local computing device 106).

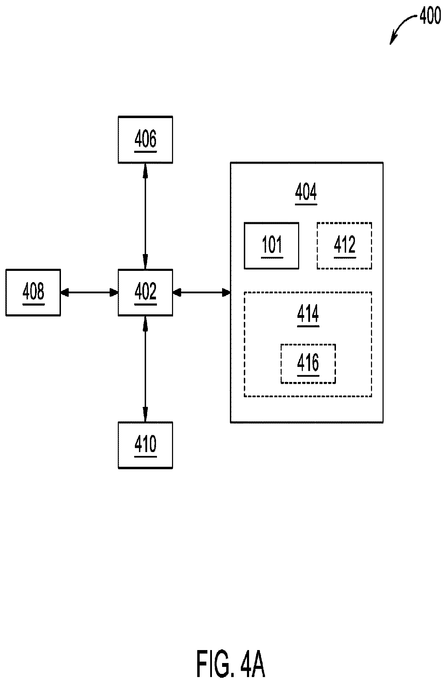

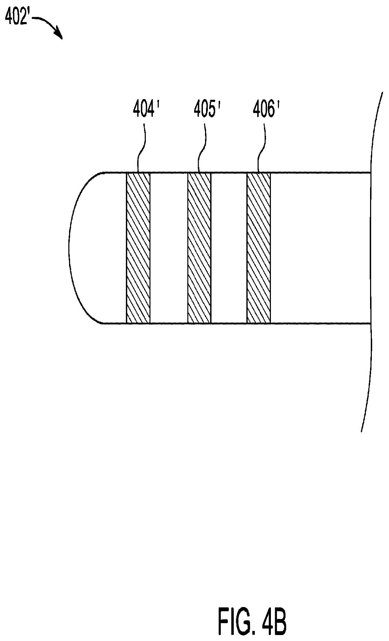

FIG. 4A is a block diagram of an example device 400 in which one or more features of the disclosure can be implemented. The device 400 may be local computing device 106, for example. The device 400 can include, for example, a computer, a gaming device, a handheld device, a set-top box, a television, a mobile phone, or a tablet computer. The device 400 includes a processor 402, a memory 404, a storage device 406, one or more input devices 408, and one or more output devices 410. The device 400 can also optionally include an input driver 412 and an output driver 414. It is understood that the device 400 can include additional components not shown in FIG. 4 including an artificial intelligence accelerator.

In various alternatives, the processor 402 includes a central processing unit (CPU), a graphics processing unit (GPU), a CPU and GPU located on the same die, or one or more processor cores, wherein each processor core can be a CPU or a GPU. In various alternatives, the memory 404 is located on the same die as the processor 402, or is located separately from the processor 402. The memory 404 includes a volatile or non-volatile memory, for example, random access memory (RAM), dynamic RAM, or a cache. By way of example, the map revision techniques described herein are implemented as a processor executable code or software that can be stored on the memory 404 (as shown) and executed by processor 402, and rooted in process operations by, and in processing hardware of, the example device 400.

The storage device 406 includes a fixed or removable storage means, for example, a hard disk drive, a solid-state drive, an optical disk, or a flash drive. The input devices 408 include, without limitation, a keyboard, a keypad, a touch screen, a touch pad, a detector, a microphone, an accelerometer, a gyroscope, a biometric scanner, or a network connection (e.g., a wireless local area network card for transmission and/or reception of wireless IEEE 802 signals). The output devices 410 include, without limitation, a display device, a speaker, a printer, a haptic feedback device, one or more lights, an antenna, or a network connection (e.g., a wireless local area network card for transmission and/or reception of wireless IEEE 802 signals).

The input driver 412 communicates with the processor 402 and the input devices 408, and permits the processor 402 to receive input from the input devices 408. The output driver 414 communicates with the processor 402 and the output devices 410, and permits the processor 402 to send output to the output devices 410. It is noted that the input driver 412 and the output driver 414 are optional components, and that the device 400 will operate in the same manner if the input driver 412 and the output driver 414 are not present. The output driver 414 includes an accelerated processing device ("APD") 416 which communicates to a display device as represented by the output devices 410. The APD 416 accepts compute commands and graphics rendering commands from processor 402, processes those compute and graphics rendering commands, and provides pixel output to display device for display. As described in further detail below, the APD 416 includes one or more parallel processing units to perform computations in accordance with a single-instruction-multiple-data ("SIMD") paradigm. Thus, although various functionality is described herein as being performed by or in conjunction with the APD 416, in various alternatives, the functionality described as being performed by the APD 416 is additionally or alternatively performed by other computing devices having similar capabilities that are not driven by a host processor (e.g., processor 402) and provides graphical output to a display device. For example, it is contemplated that any processing system that performs processing tasks in accordance with a SIMD paradigm may perform the functionality described herein. Alternatively, it is contemplated that computing systems that do not perform processing tasks in accordance with a SIMD paradigm perform the functionality described herein.

Electrical activity at a point in the heart may be typically measured by advancing a catheter containing an electrical sensor at or near its distal tip to that point in the heart, contacting the tissue with the sensor and acquiring data at that point. One drawback with mapping a cardiac chamber using a catheter containing only a single, distal tip electrode is the long period of time required to accumulate data on a point-by-point basis over the requisite number of points required for a detailed map of the chamber as a whole. Accordingly, multiple-electrode catheters have been developed to simultaneously measure electrical activity at multiple points in the heart chamber.

Multiple-electrode catheters may be implemented using any applicable shape such as a linear catheter with multiple electrodes, a balloon catheter including electrodes dispersed on multiple spines that shape the balloon, a lasso or loop catheter with multiple electrodes, a flat type multi-electrode catheter, or any other applicable shape. FIG. 4B shows an example of a linear catheter 402' including multiple electrodes 404', 405', and 406' that may be used to map a cardiac area. Linear catheter 402' may be fully or partially elastic such that it can twist, bend, and or otherwise change its shape based on received signal and/or based on application of an external force (e.g., cardiac tissue) on the linear catheter 402'.

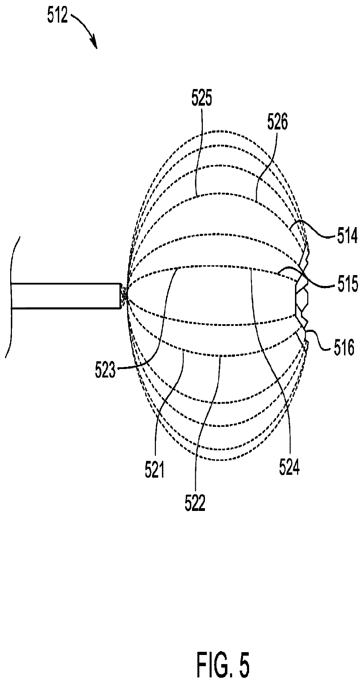

FIG. 5 shows an example of a balloon catheter 512 including multiple splines (e.g., 12 splines in the specific example of FIG. 5) including splines 514, 515, 516 and multiple electrodes on each spline including electrodes 521, 522, 523, 524, 525, and 526 as shown. The balloon catheter 512 may be designed such that when deployed into a patient's body, its electrodes may be held in intimate contact against an endocardial surface. As an example, a balloon catheter may be inserted into a lumen, such as a pulmonary vein (PV). The balloon catheter may be inserted into the PV in a deflated state such that the balloon catheter does not occupy its maximum volume while being inserted into the PV. The balloon catheter may expand while inside the PV such that electrodes on the balloon catheter are in contact with an entire circular section of the PV. Such contact with an entire circular section of the PV, or any other lumen, may enable efficient mapping and/or ablation.

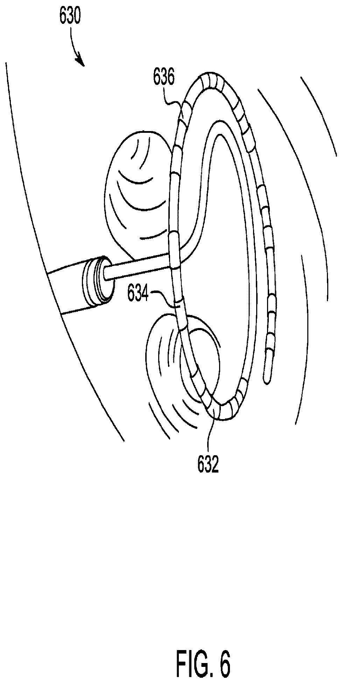

FIG. 6 shows an example of a loop catheter 630 (also referred to as a lasso catheter) including multiple electrodes 632, 634, and 636 that may be used to map a cardiac area. Loop catheter 630 may be fully or partially elastic such that it can twist, bend, and or otherwise change its shape based on received signal and/or based on application of an external force (e.g., cardiac tissue) on the loop catheter 630. While loop catheter 630 is shown as having only three electrodes, it will be understood that loop catheter can have more or fewer electrodes.

FIG. 7 shows an example of a flat type multi-electrode catheter 700 that may be used to map a cardiac area. In one example, catheter 700 includes 48 tightly-spaced (2.4 x 2.4mm, center-to-center) electrodes 702. In one example, each electrode 702 is small, on the order of 460 microns. A catheter of the type shown in Figure 7 is available from Biosense Webster, Inc., 31A Technology Drive, Irvine, CA 92618 under the name "Optrell™ Mapping Catheter."

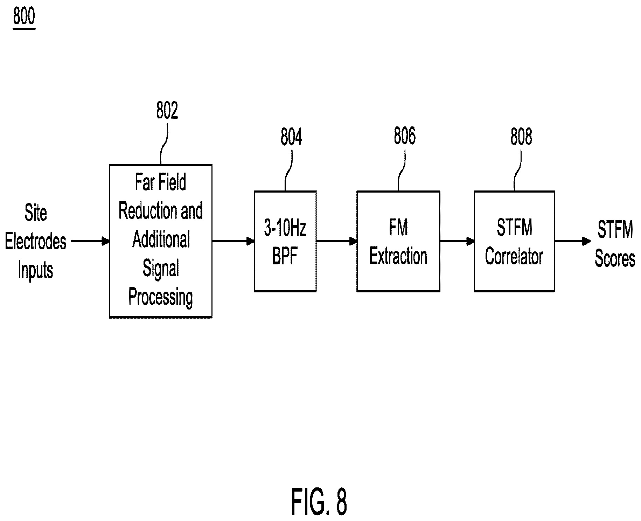

FIG. 8 depicts a method 800 for generating composite correlation scores based on electrical signals from a multi-electrode catheter, in accordance with an example. The site electrode inputs correspond to electrical measurements (or signals) collected by different electrodes in a multi-electrode catheter (e.g., electrodes 632, 634 and 636 of loop catheter 630) which are in contact with different tissue locations during a time period, e.g., when loop catheter 630 is stationary. In the example shown in Figure 8, the collected electrical signals correspond to time domain electrical signals derived from sample measurements collected from these different tissue locations during the period of time. In processing step 802, far field filtering is performed together with other signal processing on the collected signals. In one embodiment, such other signal processing includes filtering out high frequencies (e.g., filtering out frequencies from 15-400 Hz) from each of the collected signals, and converting amplitude values to absolute values. It will be understood that other signal processing techniques may also be used. In processing step 804, band pass filtering is applied to limit the signals to a frequency range of, e.g., 3-10 Hz. In processing step 806, signals output from the band pass filter are converted to frequency modulated signals (FM extraction). In the example where multi-electrode loop catheter 630 is used, the FM extraction results in three FM signals - - one for each of electrodes 632, 634 and 636. In other example, other multi-electrode catheters, including those depicted in FIGs. 4, 5 and 7, are used.

In processing step 808, correlation operations are performed to generate composite correlation scores (referred to in the diagram as "STFM scores" where "STFM" is short for spatial-temporal frequency modulated) corresponding to the spatial locations of each of the three electrodes in the heart at the time the site electrode inputs were collected. For purposes of illustration in this example, the three FM signals resulting from the FM extraction -- one for each of electrodes 632, 634 and 636 -- will be referred to as signals $A_1$, $A_2$ and $A_3$, respectively. Referring first to signal $A_1$, in one example, an individual linear correlation between signal $A_1$ and signal $A_2$ ($Corr(A_1, A_2)$) is determined, and an individual linear correlation between signal $A_1$ and $A_3$ ($Corr(A_1, A_3)$) is determined. The composite correlation score for $A_1$ ($Score(A_1)$) is then determined from the individual correlation values ($Corr(A_1, A_2)$ and $Corr(A_1, A_3)$). In a specific example, the composite correlation score for $A_1$ ($Score(A_1)$) is the average of individual correlation values ($Corr(A_1, A_2)$ and $Corr(A_1, A_3)$). A similar process is then repeated to determine composite correlation scores for signals $A_2$ and $A_3$. For example, the composite correlation score for signal $A_2$ ($Score(A_2)$) is determined from the correlation between $A_1$ and $A_2$ ($Corr(A_1, A_2)$ and the correlation between $A_2$ and $A_3$ $Corr(A_2, A_3)$). In a specific example, the composite correlation score for $A_2$ ($Score(A_2)$) is the average of individual correlation values ($Corr(A_1, A_2)$ and $Corr(A_2, A_3)$). Similarly, the composite correlation score for signal $A_3$ ($Score(A_3)$) is determined from the correlation between $A_1$ and $A_3$ ($Corr(A_1, A_3)$ and the correlation between $A_2$ and $A_3$ $Corr(A_2, A_3)$). In the specific example, the composite correlation score for $A_3$ ($Score(A_3)$) is the average of individual correlation values ($Corr(A_1, A_3)$ and $Corr(A_2, A_3)$). From a spatial perspective, $Score(A_1)$, $Score(A_2)$ and $Score(A_3)$ are each associated with the respective locations in the heart of electrodes 632, 634 and 636 at the time the site electrode inputs were collected.

As noted above, the multi-electrode catheter used for the techniques described herein is not limited to a 3-electrode catheter. The above example can be generalized to a case where the multi-electrode catheter has m electrodes (where m is an integer that is two or more). In specific embodiments, a weighting function is applied to determine the composite correlation score for each electrode. Below is an exemplary formula for determining the composite correlation score for each electrode using a weighting function:

$$Score(A_i) = \frac{\sum_{j=1}^{m} w_{ij} Corr(A_i, A_j)}{\sum_{j=1}^{m} w_{ij}}$$

In the exemplary formula above, the weight ($w_{ij}$) is determined based on a distance between electrodes i and j (also denoted as "distance $_{ij}$") based on the following conditions:

$$\text{If (distance}_{ij}) < c_1, \text{ then } w_{ij} = 0$$

$$\text{If (distance}_{ij}) \geq c_1, \text{ then } w_{ij} = e^{-\frac{distance_{ij}}{2\sigma^2}}$$

$c_1$ = 3mm (minimal distance to consider proximate electrodes)
$\sigma$ = 10mm (effective diameter to average electrodes)

Where, $c_1$ corresponds to a minimum distance required for electrodes i and j to be considered proximate, and $\sigma$ corresponds to an effective average diameter of the electrodes. In a specific example, $c_1$ and $\sigma$ are 3mm and 10mm, respectively.

While in some examples, the composite correlation score is a simple average of the individual correlation scores for a given electrode, it will be understood that other functions can be used to generate the composite correlation score for the individual correlation scores. In addition, while in the example above each individual correlation score corresponded to a linear correlation between two signals, it will be understood that other correlation functions can be used to determine the individual correlation values described herein.

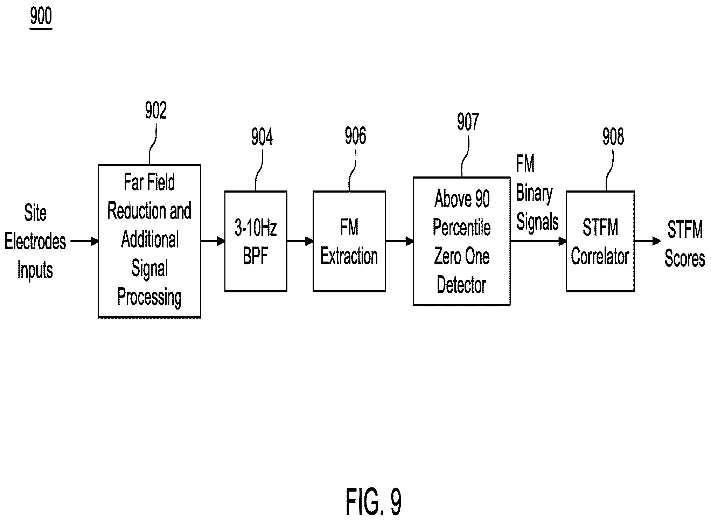

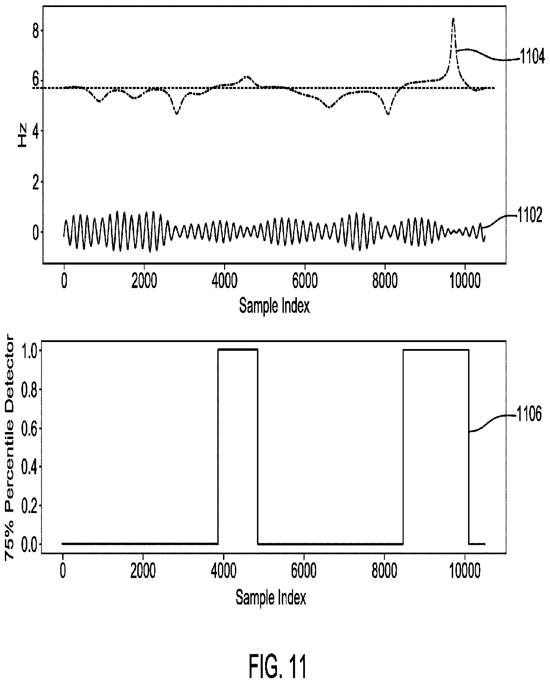

FIG. 9 depicts an embodiment of a technique 900 for generating composite correlation scores based on electrical signals from a multi-electrode catheter, in accordance with a further example. In FIG. 9, operations 902, 904, 906 and 908 are substantially the same as operations 802, 804, 806 and 808, respectively, described above in connection with FIG. 8. However, in FIG. 9, operation 907 is used to convert the output of the FM extractor to a binary signal prior to performing the correlation operations in step 908. It will be understood that such binary conversion is optional and not required. In one example, discussed more fully in connection with FIG. 11 below, the binary conversion operation converts portions of the extracted FM signal above the 90th percentile to a "1" and portions of the signal below that percentile to a "0." It will be understood that other threshold levels could be used for the binary conversion.

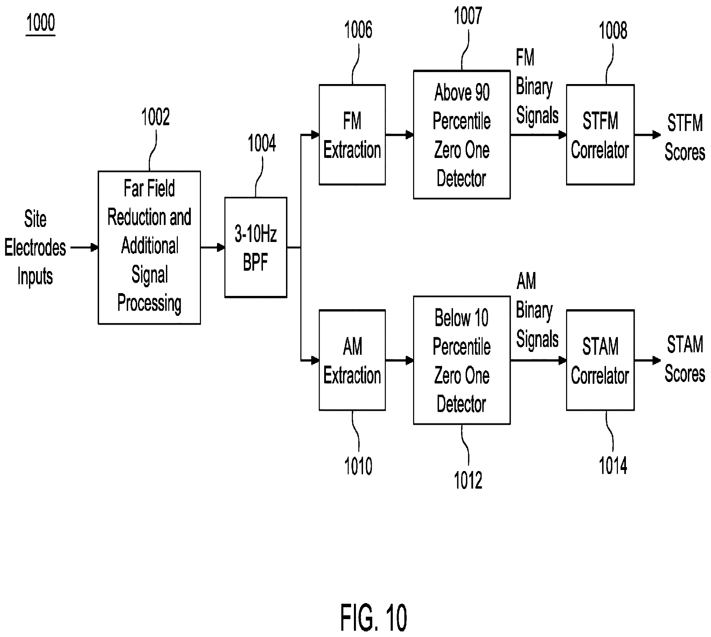

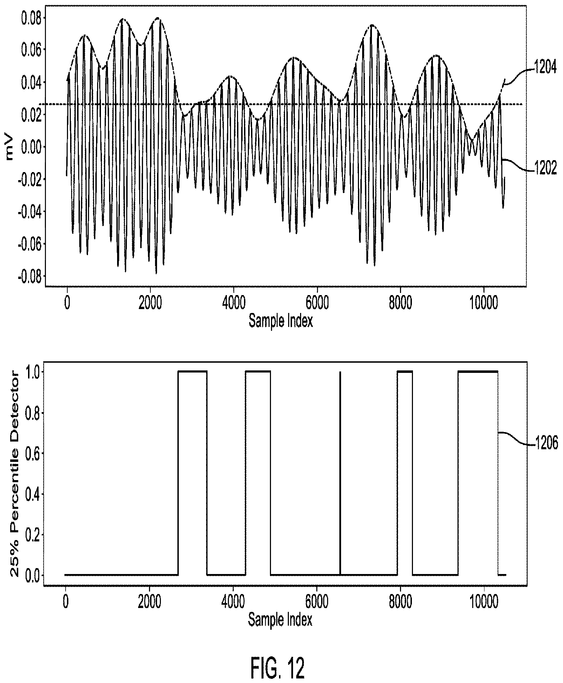

FIG. 10 depicts an embodiment of a technique 1000 for generating composite correlation scores based on electrical signals from a multi-electrode catheter, in accordance with a still further example. In FIG. 10, operations 1002, 1004, 1006 and 1008 are substantially the same as operations 802, 804, 806 and 808, respectively, described above in connection with FIG. 8; and operation 1007 is substantially the same as operation 907 described above in connection with FIG. 9. The process of FIG. 10 further includes amplitude modulation (AM) extraction operation 1010 where signals output from the band pass filter are converted to amplitude modulated signals (AM extraction). Continuing with the example from FIG. 8 where multi-electrode loop catheter 630 is used, the AM extraction results in three AM signals -- one for each of electrodes 632, 634 and 636. Operation 1012 is used to convert the output of the AM extractor to a binary signal prior to performing the correlation operations in step 1014. In one example, discussed more fully in connection with FIG. 12 the below, the binary conversion operation converts portions of the extracted AM signal above the 90th percentile to a "1" and portion of the signal below that percentile to a "0." It will be understood that other threshold levels could be used for the binary conversion. In some embodiments, operation 1012 is omitted and the output of the AM extraction operation 1010 is used for correlation operation 1014.

In processing step 1014, correlation operations are performed to generate composite correlation scores (referred to in the diagram as "STAM scores" where "STAM" is short for spatial-temporal amplitude modulated) corresponding to the spatial locations of each of the three electrodes in the heart at the time site electrode inputs were collected. Correlation operation 1014 is performed in substantially the same way as operation 808, with the exception that the inputs to the correlation operation correspond to AM signals. Continuing with the same example, from a spatial perspective, each of the composite correlation scores (referred to in the diagram as STAM scores) are associated with the respective location in the heart of electrodes 632, 634 and 636 at the time the site electrode inputs were collected. As noted above in connection with FIG. 8, the multi-electrode catheter used for the techniques described herein is not limited to a 3-electrode catheter, but may be generalized to a case where the multi-electrode catheter has i electrodes. In addition, while in the above example, the composite correlation score (STAM score) is simply an average of the individual correlation scores for a given electrode, it will be understood that other functions can be used to generate the composite correlation score for the individual correlation scores.

FIG. 11 illustrates generation of an FM binary signal 1106 based on a frequency modulated signal 1104 associated with a catheter electrode, according to an exemplary implementation of the process shown in FIG. 9. Time-domain signal 1102 corresponds to a 3-10 Hz signal output from band pass filter operation 904. FM signal 1104 corresponds to the FM signal extracted by FM extractor operation 906. In the example shown, binary conversion operation 907 converts portions of the extracted FM signal 1104 above the 90th percentile to a "1" and portions of the signal below that percentile to a "0." Again, it will be understood that other threshold levels could be used for the binary conversion.

FIG. 12 illustrates generation of an AM binary signal 1206 based on an amplitude modulated signal derived from a catheter electrode, according to an exemplary implementation of operations 1010 and 1012 shown in FIG. 10. Time-domain signal 1202 corresponds to a 3-10 Hz signal output from band pass filter operation 1004. AM signal 1202 corresponds to the AM signal extracted by AM extractor operation 1010. In the example shown, binary conversion operation 1012 converts portions of the extracted AM signal 1204 above the 90th percentile to a "1" and portions of the signal below that percentile to a "0." Again, other threshold levels could be used for the binary conversion.

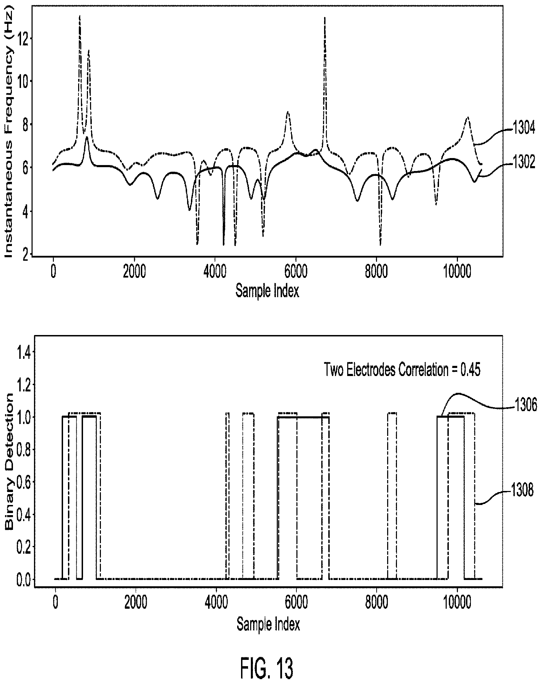

FIG. 13 illustrates a technique for identifying an individual correlation value based on FM signals derived from two different electrodes of a multi-catheter electrode, according to an exemplary implementation of correlation operation 908 in FIG. 9. In connection with the example used above for describing FIG. 9, correlation operation 908 determined a linear correlation between signals $A_1$ and $A_2$ (also referred to above as (Corr($A_1$, $A_2$)), where $A_1$ and $A_2$ corresponded to binary signals output by operation 907. In the example of Figure 13, signal 1302 corresponds an extracted FM signal associated with an input signal from one electrode (e.g., electrode 632) and signal 1304 corresponds to an extracted FM signal associated with an input signal from a different electrode (e.g., electrode 634) during a given time period. Binary conversion operation 907 converts extracted FM signal 1302 to binary signal 1306, and extracted FM signal 1304 to binary signal 1308. To determine Corr($A_1$, $A_2$), correlation operation 908 determines a linear correlation between binary signals 1306 and 1308, which in the example shown is equal to 0.45.

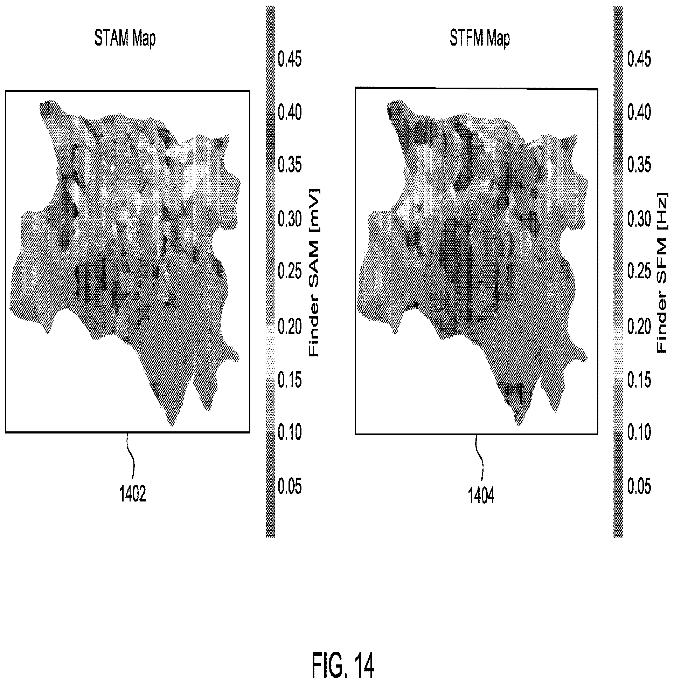

Figure 14 illustrates examples of electro-anatomical maps depicting composite correlation scores determined for multiple spatial locations in a heart, according to examples. In the exemplary maps, greyscaling is used to spatially depict the composite correlation scores determined using the techniques described above at various locations in the heart. For example, the composite correlation scores depicted in map 1402 correspond to STAM scores determined by operation 1014 based on AM signals, and the composite correlation scores depicted in map 1404 correspond to STFM scores determined by operation 1008 based on FM signals. Generally, the higher the composite correlation score for a given spatial location, the more likely it is that the spatial location corresponds to a suspected trigger of AF. In some embodiments, a threshold may be used to identify suspected AF triggers. For example, one or both of maps 1402 and 1404 may visually categorize spatial locations having a composite correlation at or above 0.35 or 0.40 as corresponding to suspected AF triggers. In some embodiments, only one of maps 1402 and 1404 is used to identify (for ablation) spatial locations that are suspected AF triggers. In other embodiments, both maps are used together to identify AF trigger locations for ablation. In one such embodiment, map 1404 (based on FM signals) is used to initially identify potential AF triggers, and map 1402 (based on AM signals) is used as confirmation that a given spatial location corresponds to an AF trigger before proceeding with ablation of the spatial location.

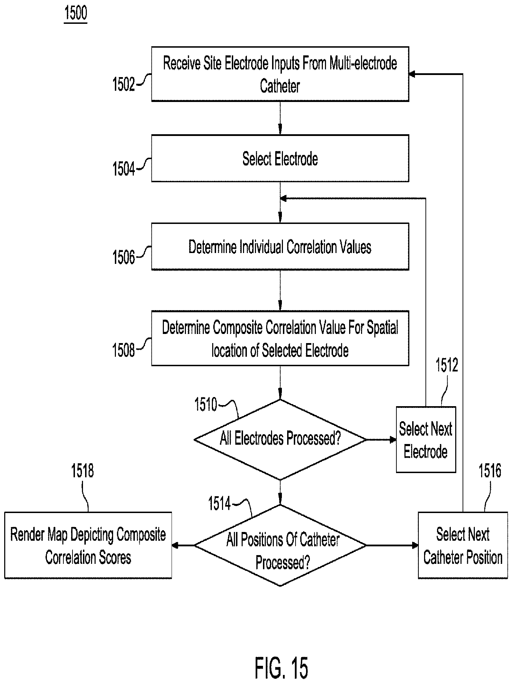

FIG. 15 depicts a method 1500 according to one or more embodiments. In step 1502, electrodes on a multi-electrode catheter provide site specific input signals. In one example, the site electrode inputs correspond to electrical sample measurements (or signals) collected by different electrodes in a multi-electrode catheter (e.g., electrodes 632, 634 and 636 of loop catheter 630) which are in contact with different tissue locations during a time period, e.g., when loop catheter 630 is stationary. In some examples, further processing is applied to the site specific input signals. In one example, far field filtering is performed together with other signal processing on the site specific input signals. In one embodiment, such other signal processing includes filtering out high frequencies (e.g., filtering out frequencies from 15-400 Hz) from each of the input signals, converting amplitude values to absolute values, and the applying band pass filtering to limit the signals to a frequency range of, e.g., 3-10 Hz. In some examples, signals output from the band pass filter are converted to FM or AM signals (or both). In some examples, the FM and/or AM signals are further converted into binary signals.

In step 1504, one of the electrodes from which the site specific input signals were received in step 1502 is selected for correlation processing. In step 1506, an individual correlation value (e.g., a linear correlation value) is determined between a signal associated with the selected electrode (e.g., a processed signal associated with the selected electrode) and a signal associated with each of the other ones of the plurality of electrodes from which site specific input signals were received in step 1502. In step 1508, a composite correlation value for the site of the selected electrode is determined from the individual correlation values determined in step 1506. In a specific example, the composite correlation value is an average of the individual correlation values. Steps 1510 and 1512 illustrate that a composite correlation score is determined using the same process for each of the other electrodes in the catheter from which the site specific input signals were received in step 1502. Steps 1514 and 1516 signify that the process reflected in steps 1502-1512 is repeated as the catheter is moved to various locations within the heart during a medical procedure (e.g., an ablation procedure).

In step 1518, an electro-anatomical map of the heart is rendered for display. The map spatially depicts information representative of the composite correlation scores determined for various locations in the heart using steps 1502-1516. The information representative of the composite correlation scores displayed on the electro-anatomical map indicates one or more spatial locations that correspond to a suspected trigger of AF.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the blocks may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

Although features and elements are described above in particular combinations, one of ordinary skill in the art will appreciate that each feature or element can be used alone or in any combination with the other features and elements. In addition, the methods described herein may be implemented in a computer program, software, or firmware incorporated in a computer-readable medium for execution by a computer or processor. A computer readable medium, as used herein, is not to be construed as being transitory signals per se, for example radio waves or other freely propagating electromagnetic waves, electromagnetic waves propagating through a waveguide or other transmission media (e.g., light pulses passing through a fiber-optic cable), or electrical signals transmitted through a wire

Examples of computer-readable media include electrical signals (transmitted over wired or wireless connections) and computer-readable storage media. Examples of computer-readable storage media include, but are not limited to, a register, cache memory, semiconductor memory devices, magnetic media (e.g., internal hard disks and removable disks), magneto-optical media, optical media (e.g., compact disks (CD) and digital versatile disks (DVDs)), a random access memory (RAM), a read-only memory (ROM), an erasable programmable read-only memory (EPROM or Flash memory), a static random access memory (SRAM), and a memory stick. A processor in association with software may be used to implement a radio frequency transceiver for use in a terminal, base station, or any host computer.

The terminology used herein is for the purpose of describing particular embodiments only and is not intended to be limiting. As used herein, the singular forms "a", "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one more other features, integers, steps, operations, element components, and/or groups thereof.

The descriptions of the various embodiments herein have been presented for purposes of illustration, but are not intended to be exhaustive or limited to the embodiments disclosed. Many modifications and variations will be apparent to those of ordinary skill in the art without departing from the scope and spirit of the described embodiments. The terminology used herein was chosen to best explain the principles of the embodiments, the practical application or technical improvement over technologies found in the marketplace, or to enable others of ordinary skill in the art to understand the embodiments disclosed herein.

**Claims**

1. A method for identifying one or more spatial locations that correspond to suspected triggers of atrial fibrillation in a human heart, wherein the method is performed using a catheter having a plurality of electrodes configured to be positioned in the heart, and the electrodes configured to collect electrical signals from spatial locations within the heart as the catheter moves within the heart during a medical procedure, the method comprising:

   determining at least one composite correlation score associated with a selected spatial location in the heart, wherein the at least one composite correlation score is based on a plurality of individual correlation values, wherein each individual correlation value is based on a correlation between at least one signal associated with a selected electrode positioned at the selected spatial location and at least one signal associated with another one of the plurality of electrodes when the selected electrode is positioned at the selected spatial location;
   determining at least one composite correlation score associated with each of a plurality of other selected spatial locations in the heart by repeating said determining for each of the other selected spatial locations;
   displaying an electro-anatomical map of the heart, wherein the map spatially depicts information representative of the composite correlation score determined for each of the selected spatial locations; and
   wherein the information representative of the composite correlation score displayed on the electro-anatomical map for at least one of the selected spatial locations indicates that the at least one selected spatial location corresponds to a suspected trigger of atrial fibrillation.

2. The method of claim 1, wherein each individual correlation value is based on a correlation between a first frequency modulated signal associated with the selected electrode positioned at the selected spatial location and a second frequency modulated signal associated with another one of the plurality of electrodes when the selected electrode is positioned at the selected spatial location.

3. The method of claim 1, wherein the at least one composite correlation score corresponds to an average of the plurality of individual correlation values.

4. The method of claim 3, wherein each individual correlation value is also based on a correlation between a first amplitude modulated signal associated with the selected electrode positioned at the selected spatial location and a second amplitude modulated signal associated with another one of the plurality of electrodes when the selected electrode is positioned at the selected spatial location.

5. The method of claim 1, further comprising:

displaying a first electro-anatomical map of the heart, wherein the first map spatially depicts information representative of a first composite correlation score determined for each of the selected spatial locations; wherein the first composite correlation score for each of the selected spatial locations is based on a plurality of first individual correlation values each of which is based on a correlation between frequency modulated signals; and displaying a second electro-anatomical map of the heart, wherein the second map spatially depicts information representative of a second composite correlation score determined for each of the selected spatial locations; wherein the second composite correlation score for each of the selected spatial locations is based on a plurality of second individual correlation values each of which is based on a correlation between amplitude modulated signals.

6. The method of claim 1, wherein the at least one signal derived from the selected electrode positioned at the selected spatial location is generated by collecting a first electrical signal from the selected electrode, and applying at least far field reduction processing and band pass filtering to the first electrical signal.

7. The method of claim 1, wherein the at least one signal derived from the selected electrode positioned at the selected spatial location is generated by collecting a first electrical signal from the selected electrode, applying signal processing to the first electrical signal to generate a second electrical signal, and converting the second electrical signal into a binary signal.

8. The method of claim 1, wherein each individual correlation value is based on a linear correlation between the at least one signal associated with the selected electrode positioned at the selected spatial location and the at least one signal associated with another one of the plurality of electrodes when the selected electrode is positioned at the selected spatial location.

9. The method of claim 1, wherein the information representative of the composite correlation score displayed on the electro-anatomical map for at least one of the selected spatial locations indicates that the at least one selected spatial location corresponds to a suspected trigger of atrial fibrillation if the spatial position has a corresponding composite correlation score that is above a threshold.

10. A system for identifying one or more spatial locations that correspond to suspected triggers of atrial fibrillation in a heart of a patient, comprising:

a catheter having a plurality of electrodes,
a processor communicatively coupled to memory and the electrodes,
a display communicatively coupled to the processor,
the processor operating to:

receive electrical signals from spatial locations within the heart as the catheter moves within the heart during a medical procedure;
for each of a plurality of selected spatial locations in the heart, determine at least one composite correlation score associated with the selected spatial location, wherein the at least one composite correlation score is based on a plurality of individual correlation values, wherein each individual correlation value is based on a correlation between at least one signal associated with a selected electrode positioned at the selected spatial location and at least one signal associated with another one of the plurality of electrodes when the selected electrode is positioned at the selected spatial location;
cause an electro-anatomical map of the heart to be displayed on the display, wherein the map spatially depicts information representative of the composite correlation score determined for each of the selected spatial locations; and

wherein the information representative of the composite correlation score displayed on the electro-anatomical map for at least one of the selected spatial locations indicates that the at least one selected spatial location corresponds to a suspected trigger of atrial fibrillation.

11. The system of claim 10, wherein each individual correlation value is based on a correlation between a first frequency modulated signal associated with the selected electrode positioned at the selected spatial location and a second frequency modulated signal associated with another one of the plurality of electrodes when the selected electrode is positioned at the selected spatial location.

12. The system of claim 10, wherein the at least one composite correlation score corresponds to an average of the plurality of individual correlation values, and also based on a correlation between a first amplitude modulated signal associated with the selected electrode positioned at the selected spatial location and a second amplitude modulated signal associated with another one of the plurality of electrodes when the selected electrode is positioned at the selected spatial location.

13. The system of claim 10, the processor operating to:

cause a first electro-anatomical map of the heart to be displayed on the display, wherein the first map spatially depicts information representative of a first composite correlation score determined for each of the selected spatial locations; wherein the first composite correlation score for each of the selected spatial locations is based on a plurality of first individual correlation values each of which is based on a correlation between frequency modulated signals; and

cause a second electro-anatomical map of the heart to be displayed on the display, wherein the second map spatially depicts information representative of a second composite correlation score determined for each of the selected spatial locations; wherein the second composite correlation score for each of the selected spatial locations is based on a plurality of second individual correlation values each of which is based on a correlation between amplitude modulated signals.

14. The system of claim 10, wherein the at least one signal associated with the selected electrode positioned at the selected spatial location is generated by collecting a first electrical signal from the selected electrode, and applying at least far field reduction processing and band pass filtering to the first electrical signal; or by collecting a first electrical signal from the selected electrode, applying signal processing to the first electrical signal to generate a second electrical signal, and converting the second electrical signal into a binary signal.

15. The system of claim 10, wherein:

each individual correlation value is based on a linear correlation between the at least one signal associated with the selected electrode positioned at the selected spatial location and the at least one signal associated with another one of the plurality of electrodes when the selected electrode is positioned at the selected spatial location; and

wherein the information representative of the composite correlation score displayed on the electro-anatomical map for at least one of the selected spatial locations indicates that the at least one selected spatial location corresponds to a suspected trigger of atrial fibrillation if the spatial position has a corresponding composite correlation score that is above a threshold.

FIG. 1

FIG. 2

FIG. 3

FIG. 4A

FIG. 4B

FIG. 5

FIG. 6

FIG. 7

800

FIG. 8

EP 4 721 664 A1

900

Site
Electrodes
Inputs

902

Far Field
Reduction and
Additional
Signal
Processing

904

3-10Hz
BPF

906

FM
Extraction

907

Above 90
Percentile
Zero One
Detector

FM
Binary
Signals

908

STFM
Correlator

STFM
Scores

FIG. 9

EP 4 721 664 A1

1000

Site
Electrodes
Inputs
→ Far Field
Reduction and
Additional
Signal
Processing
`1002`

→ 3-10Hz
BPF
`1004`

→ FM
Extraction
`1006`
→ Above 90
Percentile
Zero One
Detector
`1007`
FM
Binary
Signals
→ STFM
Correlator
`1008`
→ STFM
Scores

→ AM
Extraction
`1010`
→ Below 10
Percentile
Zero One
Detector
`1012`
AM
Binary
Signals
→ STAM
Correlator
`1014`
→ STAM
Scores

FIG. 10

FIG. 11

FIG. 12

FIG. 13

STFM Map

Finder SFM [Hz]

0.45
0.40
0.35
0.30
0.25
0.20
0.15
0.10
0.05

1404

STAM Map

Finder SAM [mV]

0.45
0.40
0.35
0.30
0.25
0.20
0.15
0.10
0.05

1402

FIG. 14

FIG. 15

# EUROPEAN SEARCH REPORT

Application Number

EP 25 20 5530

Europäisches
Patentamt
European
Patent Office
Office européen
des brevets

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2014/235988 A1 (GHOSH SUBHAM [IN]) 21 August 2014 (2014-08-21) * paragraphs [0024] - [0042], [0067] - [0094] * * figures 1-11 * | 1-15 | INV. A61B5/287 A61B5/361 A61B5/00 |
| A | US 2021/338137 A1 (GARCÍA QUINTANILLA JORGE [ES] ET AL) 4 November 2021 (2021-11-04) * paragraphs [0026] - [0044], [0164] - [0215] * | 1-15 | |
| A | QUINTANILLA JORGE G. ET AL: "Instantaneous Amplitude and Frequency Modulations Detect the Footprint of Rotational Activity and Reveal Stable Driver Regions as Targets for Persistent Atrial Fibrillation Ablation", CIRCULATION RESEARCH, vol. 125, no. 6, 30 August 2019 (2019-08-30), pages 609-627, XP093366533, US ISSN: 0009-7330, DOI: 10.1161/CIRCRESAHA.119.314930 * the whole document * | 1-15 | TECHNICAL FIELDS SEARCHED (IPC)  A61B |

The present search report has been drawn up for all claims

| Place of search | Date of completion of the search | Examiner |
|---|---|---|
| The Hague | 25 February 2026 | Athanasiadis, I |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

 

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 25 20 5530

This annex lists the patent family members relating to the patent documents cited in the above-mentioned European search report.
The members are as contained in the European Patent Office EDP file on
The European Patent Office is in no way liable for these particulars which are merely given for the purpose of information.

25-02-2026

| Patent document cited in search report | | | Publication date | Patent family member(s) | | | Publication date |
|---|---|---|---|---|---|---|---|
| US 2014235988 | A1 | | 21-08-2014 | CN | 105072982 | A | 18-11-2015 |
| | | | | EP | 2958484 | A1 | 30-12-2015 |
| | | | | US | 2014235988 | A1 | 21-08-2014 |
| | | | | US | 2015223863 | A1 | 13-08-2015 |
| | | | | WO | 2014130169 | A1 | 28-08-2014 |
| US 2021338137 | A1 | | 04-11-2021 | EP | 3636147 | A1 | 15-04-2020 |
| | | | | EP | 3863510 | A1 | 18-08-2021 |
| | | | | ES | 2968527 | T3 | 10-05-2024 |
| | | | | US | 2021338137 | A1 | 04-11-2021 |
| | | | | WO | 2020074712 | A1 | 16-04-2020 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 55391199 B
- US 5443489 A
- US 5558091 A
- US 6172499 B
- US 6239724 B
- US 6332089 B
- US 6484118 B
- US 6618612 B
- US 6690963 B
- US 6788967 B
- US 6892091 B
- US 7536218 B
- US 7756576 B
- US 7848787 B
- US 7869865 B
- US 8456182 B